# EUROPEAN PATENT SPECIFICATION

(11) **EP 2 155 144 B1**
(45) Date of publication and mention of the grant of the patent: **20.11.2013**
(21) Application number: 07720119.2
(22) Date of filing: 04.05.2007
(51) Int. Cl.: A61Q 11/00, A61K 8/58

(54) **ORAL COMPOSITION**
ORALE ZUSAMMENSETZUNG
COMPOSITION ORALE

(43) Date of publication of application: 24.02.2010
(73) Proprietor: Swissdent Cosmetics AG, 8001 Zürich (CH)
(72) Inventor: VELKOBORSKY, Vaclav, CH-8008 Zürich (CH); GHOSH, Sankha, CH-8001 Zürich (CH)
(74) Representative: Grimm, Siegfried
(86) International application number: PCT/CH2007/000222
(87) International publication number: WO 2008/134900

(56) References cited:
- WO-A-2006/106366
- FR-A- 2 796 383
- US-A- 4 764 497
- DATABASE WPI Week 199226 Derwent Publications Ltd., London, GB; AN 1992-214194 XP002463425 & JP 04 144918 A (SHOWA DENKO KK) 19 May 1992 (1992-05-19)

## Description

The invention relates to titanium alkoxides as pharmaceuticals, the use of titanium alkoxides and compositions containing titanium alkoxides for oral care, in particular for the treatment of disorders of the teeth and/or cosmetic applications to the teeth. It is believed that titanium alkoxides transform into nano ceramic titanium dioxide upon application to the teeth, which provides a synthetic route towards glazing the enamel surface.

A tooth is comprised of an inner dentin layer and an outer hard enamel layer that is the protective layer of the tooth. The enamel layer of a tooth is naturally an opaque white or slightly off-white color. It is this enamel layer that can become largely stained or discolored. The enamel layer of a tooth is composed of hydroxyapatite (HCA) mineral crystals that create a somewhat porous surface. It is believed that this porous nature of the enamel layer is what allows staining agents and discoloring substances to permeate the enamel and discolor the tooth. Many substances that a person confronts or comes in contact with on a daily basis may "stain" or reduce the "whiteness" of one's teeth. In particular consume foods, tobacco products and liquids (such as tea and coffee) tend to stain one's teeth. These products or substances tend to accumulate on the enamel layer of the tooth and form a film over the teeth. These staining and discoloring substances can then permeate the enamel layer. This problem occurs gradually over many years, but imparts a noticeable discoloration of the enamel of one's teeth.

To address this issue, a number of approaches are known. U.S. 5,256,402 and U.S. 6,174,516 disclose peroxide containing dentifrice compositions and its application (e.g. by brushing the teeth). The aqueous dentifrice compositions disclosed therein contain a urea or hydrogen peroxide compound, an alkali pyrophosphate salt and an abrasive material. Although such peroxide dentifrice compositions of the prior art are claimed to be effective in whitening teeth, the concentration below 10%w of peroxides content that, which is legally allowed in cosmetic dentistry, is not effective in whitening the teeth, which are especially highly discoloured. Furthermore, the low PH of peroxide dentifrice causes acid dissolution of HCA, and the high relative dental abrasion (RDA) that stems from the abrasive materials stipulates mechanical damage to the enamel surface. In addition, two side effects that occur most often are an increase in tooth sensitivity by irritating the pulp, and oxidative irritation of the soft tissues of the mouth, particularly the gums and gingeva. In summary, these compositions are little effective, difficult to apply, and have unfavorable side effects.

An alternative approach to address this issue is disclosed in U.S. 5,735,942 and U.S. 6,086,374. These documents suggest a biomimetic mineralizing of enamel and dentin through the application of bioactive glass materials, as by brushing, to the teeth. The non-aqueous dentifrice compositions disclosed therein contain a complex adduct of Calcium Sodium Phosphosilicate particles as an active ingredient, and an abrasive material. It is suggested that bioglass particles undergo a hydrolysis reaction during contact to the saliva water, as when brushing the teeth, to produce sodium, calcium, silicate and phosphate ions, followed by an alkaline PH induced nucleation of Calcium Phosphate grains which, onto dentin and enamel, finally form HCA. Although such bio-mineralizing compositions of the prior art are claimed to be effective in occluding the enamel tubules and reviving the HCA, the disclosed process is rather slow and little effective because the underlying hydrolysis of bioglass materials and subsequent formation of HCA are eventually very slow in oral environment. Furthermore, the prior art suffers from the acid dissolution of Calcium Phosphate grains and HCA induced by the organic acids that stems from the oral plaque. The produced Calcium Phosphate layers as well as HCA in the prior art does not offer a protection towards further absorption of colored pigments, and are neither effective in masking the colored pigments onto enamel nor effective in enhancing the whiteness of the teeth. Thus, the prior art does not furnish an effective means for the tooth whitening.

Further, ceramic materials are well known in industry and have played a key role in the advancement of material science. In particular, the development of ceramic crowns and implants is considered a huge stride in oral care. Also, ceramic thin films and coatings are known in many fields, e.g. in bone implants and wear-resistant coatings. It is also known that conventional ceramic processing, cannot be easily implemented for surface coatings. Film cracks and interfacial delamination often occurs with ceramic films due to shrinkage, thermal expansion mismatch and weak guest-host van der Waals interactions. In addition, ceramic processing typically requires high temperatures and pressures for densification. Therefore, until now ceramic coating is not considered a suitable method in oral care.

Further, WO2006/106366 discloses improved oxidative hair dyes comprising titanates. FR2796383 discloses specific titanates comprising fluoride groups for pharmaceutical uses.

Thus, there is a need of compounds and compositions for oral care, in particular for whitening of teeth. It is an object of the present invention to provide such compounds and compositions and to mitigate at least some of these drawbacks of the state of the art. In particular, it is an aim of the present invention to provide a compound and a composition that has a pronounced cosmetic and / or therapeutic effect on teeth or teeth structures.

These objectives are achieved by a compound as defined in claim 1, a composition as defined in claim 3. Further aspects of the invention are disclosed in the specification and independent claims, preferred embodiments are disclosed in the specification and the dependent claims.

The present invention will be described in more detail below. It is understood that the various embodiments, preferences and ranges may be combined at will. Further, depending of the specific embodiment, selected definitions, embodiments or ranges may not apply.

Unless otherwise stated, the following definitions shall apply in this specification:

The term containing shall, in the context of this invention, also include the meaning of comprising or consisting of.

The term treatment of a disorder shall, in the context of this invention, also include the prevention and/or delay of progression of said disorder.

The term glazing is known as an art of technology in painting, to laminate an article, subject to drying, in order to raise the optical transmittance due to a change in color cast or texture (gloss or matte, for instance) of the surface. Drying time depends on the amount of medium used in the glaze; a higher ratio of medium to paint (producing a very thin, semi-transparent glaze) decreases drying time. Although glazing is a painting art it has subsequently manifested in a wide range of technologies, including Food technology, Compact oxide layer glaze in metallurgy, single and bi-layer coating in microelectronics, insulated glaze in mechanics and electrical engineering, bio-film glaze in biotechnology, ceramic glaze in photonics and fuel cell technology, and many more.

The term gloss denotes an optical property, which is based on the interaction of light with physical characteristics of a surface. It is actually the ability of a surface to reflect light into the specular direction. The factors affecting gloss are the refractive index of the material, the angle of incident light and the surface topography. Gloss can be said as a view of material appearance. Materials with smooth surfaces appear glossy, while very rough surfaces reflect no specular light and therefore appear matte. Temperature and time of drying in a glaze production play crucial role to moderate the host surface between gloss and matte.

In a first aspect, the invention relates to a compound of formula (I),

Ti(OR¹)₄ (I)

wherein R¹ represents C₁ to C₈ alkyle, as pharmaceutical.

In an advantageous embodiment, R¹ represents methyl, ethyl, n-propyl, iso-propyl, n-butyl, sec-butyl, iso-butyl, tert-butyl, n-hexyl, 2-ethyl-hexyl. In a particular advantageous embodiment, R¹ represents iso-propyl. In a further particular advantageous embodiment, R¹ represents ethyl.

Compounds of formula (I) are known and commercially available or obtainable according to known methods, e.g. by reaction of TiCl₄ with the appropriate alcohol HOR¹.

It is believed that, upon contact with water of the salvia, the compound of formula (I) forms nanostructured TiO₂ glazing the tooth. Nano structured TiO₂ exhibits interesting optical properties of. The optical properties of TiO₂ coatings strongly depend on the phase, crystallite size and porosity of the coatings. TiO₂ is a complex material because it opacifies, variegates and crystallizes glazes. Above 2% of concentration, it begins to significantly alter the glaze surface and light reflectance properties through the creation of nano-crystals. This mechanism gives soft color and pleasant opacity of the surface. Above 15%, it tends to produce an opaque and matte surface if the glaze is not overfired, although it adds spakles to the surface. On contrary, ZrO₂ coatings tend to produce more opaque and matte surface with lower reflectivity at even smaller concentration. The common method for producing TiO₂ thin coatings follows the principle of sol-gel technique using various types of alkoxides of titanium in presence of different alcohols that modify the morphology of the resulting surface. It is known that poly-alcohols stipulates rather matte surface, while ligher alcohols facilitate formation of gloss surface. Further, it is believed that TiO₂ is highly susceptible in adhering water molecules through the formation of strong Hydrogen bonding, the resulting TiO₂ surface eventually forms a further lamination of water molecules in a water rich environment, such as the oral environment. The water lamination onto TiO₂ surface further improves the overall reflectivity of the coating.

In a second aspect, the invention relates to composition containing a) one or more compounds of formula (I) as described herein; b) one or more alcohols of the formula (II)

HOR² (II)

wherein R² represents a C₁ to C₈ alkyle, C₁ to C₈ hydroxyalkyle and c) optionally pharmaceutically acceptable additives, as pharmaceutical.

Component a): Compounds of formula (I) are known, and described above. In an advantageous embodiment, the invention relates to a composition as described herein wherein component a) is present in an amount of 2 - 20% (v/v), preferably 5-15%(v/v) in particular 9-10% (v/v) of the total composition.

Component b): Alcohols of formula (II) are known and commercially available in various grades / purities. It is preferred to use water-free ("dry") alcohols. It was found that such grades improve stability of the composition as well as the cosmetic / therapeutic effect.

In an advantageous embodiment, the invention relates to a composition as described herein wherein component b) essentially consist of one alcohol.

In a further advantageous embodiment, the invention relates to a composition as described herein wherein R¹ of compound (I) and R² of compound (II) are identical. Again, it was found that this results in improved stability and improved cosmetic/therapeutic effect.

Component c) Further pharmaceutical additives may be present in a composition according to the invention. In particular viscosity modifiers, humectants, perfumes, flavours, carriers may be added. Such additives are known in the field and may be selected according to the desired formulation. Suitable viscosity modifiers are known in the field and include cellulose, pectin, xanthan, carageenan, polysorbate and its derivatives and mixtures. Suitable humemctants are known in the field and include glycerine, sorbitol, propylene glycol and its derivatives and mixtures. To further stabilize the composition, a pH value of 6 - 8, preferably 6.8 - 7.2, e.g. 7 is advantageous. Thus, suitable buffers may be added to the composition.

Suitable formulations are any liquid formulations known in the field.

In an advantageous embodiment, the invention relates to a composition containing Ti(OEt)₄ in ethanol. In a further advantageous embodiment, the invention relates to a composition containing Ti(OⁱPr)₄ in iso-propanol. In a further advantageous embodiment, the invention relates to a composition containing Ti(OⁱPr)₄ and Ti(OEt)₄ in iso-propanol / ethanol mixtures.

In an advantageous embodiment, the invention relates to a composition as described herein which is substantially free or free of peroxides.

The manufacturing of such compositions is known in the field and generally comprises mixing of the required components at room temperature. Preferably, all components are added to the stirred solvent / mixture of solvents.

It has been found, that an application 3 ml solution of 10% (v/v) titanium alkoxide in alcohol in two painting shots offers an excellent trade-off in glossing the enamel surface by ceramic layer of TiO₂ of the front line teeth upon 45 second drying via exposing on air. This is useful in the field of oral care such as, placing white pigments on the enamel, inert coating the enamel, healing the caries and micro-holes of enamel, protecting the enamel and dentin, reducing the acidic dissolution decay of HCA from enamel, overshadowing the colored pigments on enamel and dentin, preventing the absorption of colored pigments (e.g. from tar, coffee, etc.) preventing the microbial growth in the caries, tubules, etc.

In a third aspect, the invention relates to the use of a compound or composition as described herein in cosmetic applications. In particular, the invention relates to a method for effecting improved whitening of teeth or teeth structures. The compositions as described herein are stable and storable. Further, they whiten such teeth or teeth structures without damaging oral tissues when applied onto the surface of teeth.

Without being bound to theory, it is believed that laminating the teeth with nano crystalline TiO₂ provides a whitening effect of the teeth. Thus, in one embodiment, the invention relates to the use of a compound as described herein or a composition as described herein for the cosmetic treatment, in particular the whitening, of teeth or teeth structures.

It is believed that the compositions as described herein provide a novel synthetic approach towards glazing the enamel surface by nano ceramic titanium dioxide. Thus, the invention also relates to a method of glazing teeth or teeth structures by application of an effective amount of a composition as described herein and to the use of a composition as described herein for glazing of teeth or teeth structures.

In a further embodiment, the invention also relates to a method for cosmetic treatment of teeth comprising the step of contacting one or more teeth in need of such cosmetic treatment with an effective amount of a compound according to formula (I) as described herein or a composition as described herein. Such treatment preferably takes place in a single or twofold dose / a single or twofold application.

In a further advantageous embodiment, the cosmetic treatment comprises the step of a applying a single dose of 0.5 - 5 ml, e.g. 3 ml, of a composition as described above.

In a further advantageous embodiment, the invention relates to the use of a composition as described herein, in particular if formulated as a stick, for the use as a teeth make-up. Similar to the known use of lipsticks as make-up for the lips, such composition may be used as make-up for teeth. It was found that the whitening effect is visible for at least 3-5 hrs, depending on the formulation and other factors, a whitening is visible up to 15 hrs.

The cosmetic and therapeutic effects of the composition as described herein are clearly different. While a cosmetic effect is directly achieved by one single application, a therapeutic effect can only be obtained by an application over a prolonged period, e.g. daily application over one month.

The method as described herein results in an in situ synthesis of a nano-crystallite thin ceramic film of TiO2 grown on the enamel surface at room temperature through a sol-gel route. It is believed that hydrolysis of titanium alkoxide produces nano-crystallite grains of TiO2 on enamel and small amount of alcohols, which rapidly evaporate to facilitate drying and nucleation of the ceramic film. 3 ml of 10% (v/v) solution of titanium tetraisopropoxide in dry iso-propanol is considered best choice to glaze the front line ten teeth. The composition used has no hazardous and corrosive effects towards oral environment, and the solvent as well as produced isopropanol (amounting to - 3 ml) evaporate within 45 seconds, subject to exposing the teeth in air which is also beneficial to help nucleation of the nano-grains to form the coating.

It was found that a semi-transparent and semi-opaque gloss on the enamel surface upon rapid drying was obtained. The coating exhibits good coverage and whitening on the HCA enamel surface. However, this gloss is susceptible to mechanical wearing out due to its inherent brittleness. The TiO₂ coating is inert towards the chemical environment inside the mouth, and is bio-friendly in oral hygienic sense. It prevents the absorption of the pigments, sourcing from tars, wines, tea, coffee, etc., by the enamel surface and dentin.

In a fourth aspect, the invention relates to the use of a compound or composition as described herein in therapeutic applications. In particular, the invention relates to a method for the treatment of disorders of the teeth.

Without being bound to theory, it is believed that a permanent laminating of the teeth with nano crystalline TiO₂ provides an antimicrobial effect to the teeth. Thus, in one embodiment, the invention relates to the use of a compound as described herein or a composition as described herein for the therapeutic treatment, in particular an antimicrobial treatment, of teeth or teeth structures.

In one embodiment, the invention relates to the use of a compound or a composition as described herein for the manufacture of a pharmaceutical composition for the prevention, treatment, delay of progression of a disorder selected from the group consisting of tooth decay and/or tooth hypersensitivity.

In an advantageous embodiment, the invention relates to the treatment of caries. Further, the invention relates to the prevention (prophylaxis) of caries.

Thus, the invention also relates to a method for treatment of a disorder related to tooth decay and/or tooth hypersensitivity comprising the step of contacting one or more teeth in need of such treatment with an effective amount of a compound according to formula (I) or a composition as described herein. Such treatment preferably takes place in a multiple dose / a multiple application over a prolonged period of time.

In a further advantageous embodiment, the therapeutic treatment comprises the step of a applying a daily dose of 0.5 - 5 ml, e.g. 3 ml, of a composition as described above for a period of at least two months.

Thus, the invention also relates to a method for at least partly occluding dentin tubules comprising the step of contacting one or more teeth in need of such treatment with an effective amount of a compound according to formula (I) or a composition as described herein.

It is believed that during nucleation, the nano-grains of TiO₂ substitute the microbes from the caries and micro-holes on the enamel, and thus heal the enamel. Further it is believed that the TiO₂ coating ceases the contact of organic acids which are produced by the microbial respiration, such as malic acid, to the HCA on enamel, and hence preventing the decay of enamel due to acidic dissolution of HCA.

The following examples further illustrate the invention without any limitation.

### study of teeth whitening effect

General *procedure:* Tooth colour of an incisor of an arbitrary selected test person is determined by use of a Spectroshade colour measuring-tool. Within four weeks prior to the study no professional tooth cleaning took neither place, nor any whitening tooth paste was used.

Protocol: Test person's incisor was treated with a solution of titanium iso-propoxylate in iso-propanol (10 % (v/v)) by means of a foam rubber and subsequently dried on air during 45 sec. Remission, determined by Spectroshade is given in the tables below. First horizontal line indicates maximum remission. First column (10, 20, 30 ... 90) indicates values of lower reminiscence.

**Tabelle 1: Remission prior to treatment (%)**

| | 400 nm | 500 nm | 600 nm | 700 nm |
|---|---|---|---|---|
| | 31.52 | 46.62 | 57.58 | 61.02 |
| 10 | 31.94 | 48.05 | 58.49 | 61.02 |
| 20 | 33.54 | 48.99 | 59.52 | 61.02 |
| 30 | 34.76 | 49.70 | 60.30 | 61.02 |
| 40 | 36.56 | 50.10 | 60.95 | |
| 50 | 38.56 | 50.81 | 61.32 | |
| 60 | 39.96 | 51.27 | 60.92 | |
| 70 | 41.78 | 52.42 | 61.02 | |
| 80 | 43.24 | 53.63 | 61.02 | |
| 90 | 45.40 | 55.17 | 61.02 | |

**Tabelle 2: Remission one day after treatment(%)**

| | 400 nm | 500 nm | 600 nm | 700 nm |
|---|---|---|---|---|
| | 42.29 | 50.98 | 58.78 | 62.44 |
| 10 | 42.83 | 51.63 | 59.60 | 62.44 |
| 20 | 44.18 | 52.09 | 60.48 | 62.44 |
| 30 | 45.16 | 52.19 | 61.22 | 62.44 |
| 40 | 45.65 | 52.18 | 61.90 | |
| 50 | 46.14 | 52.59 | 62.32 | |
| 60 | 46.91 | 52.80 | 62.16 | |
| 70 | 47.74 | 53.92 | 62.44 | |
| 80 | 48.74 | 55.10 | 62.44 | |
| 90 | 50.23 | 56.53 | 62.44 | |

**Tabelle 3: Remission two days after treatment. (%)**

| | 400 nm | 500 nm | 600 nm | 700 nm |
|---|---|---|---|---|
| | 38.79 | 54.81 | 64.61 | 67.81 |
| 10 | 39.32 | 55.52 | 65.32 | 67.81 |
| 20 | 41.53 | 56.06 | 65.82 | 67.81 |
| 30 | 43.24 | 56.40 | 66.51 | 67.81 |
| 40 | 45.50 | 56.54 | 67.38 | |
| 50 | 48.01 | 57.12 | 67.89 | |
| 60 | 49.42 | 57.47 | 67.60 | |
| 70 | 51.18 | 58.83 | 67.81 | |
| 80 | 52.39 | 60.28 | 67.81 | |
| 90 | 54.00 | 61.97 | 67.81 | |

The treatment resulted in a immediately visible whitening of the treated tooth.

## Claims

1. A compound of formula (I),
Ti(OR¹)₄ (I)
wherein R¹ represents ethyl or iso-propyl,
for use in a method for the treatment - including prevention and delay of progression - of a disorder selected from the group consisting of tooth decay and tooth hyper-sensitivity.

2. A compound of formula (I) according to claim 1
wherein R¹ represents iso-propyl.

3. A composition containing
a) one or more compounds of formula (I) according to claim 1 or 2
b) one or more water-free alcohols of the formula HOR² wherein R² represents a C₁ to C₈ alkyle, C₁ to C₈ hydroxyalkyle,
c) optionally pharmaceutically acceptable additives,
for use in a method for the treatment -including prevention and delay of progression - of a disorder selected from the group consisting of tooth decay and tooth hyper-sensitivity.

4. A composition according to claim 3 wherein R¹ and R² are identical.

5. The compound according to claim 1, wherein said tooth decay is caries.

6. The composition according to claim 3, wherein said tooth decay is caries.

7. Use of a compound of formula (I)
Ti(OR¹)₄ (I)
wherein R¹ represents ethyl or iso-propyl,
for the cosmetic treatment of teeth, selected from the group consisting of whitening of teeth and glazing of teeth.

8. The use according to claim 7, wherein R¹ represents iso-propyl.

9. Use of a composition containing
a) one or more compounds of formula (I) according to claim 7 or 8;
b) one or more water-free alcohols of the formula HOR² wherein R² represents a C₁ to C₈ alkyle, C₁ to C₈ hydroxyalkyle,
c) optionally pharmaceutically acceptable additives,
for the cosmetic treatment of teeth, selected from the group consisting of whitening of teeth and glazing of teeth.

10. The use according to clam 9, wherein R¹ and R² are identical.

11. The use according to any of claims 7 to 9 wherein said cosmetic treatment is whitening of teeth.

12. The use according to any of claims 7 to 9 wherein said cosmetic treatment is glazing of teeth.

## Patentansprüche

1. Eine Verbindung der Formel (I),
Ti(OR¹)₄ (I)
worin R¹ Ethyl oder iso-Propyl bedeutet,
zur Verwendung in einem Verfahren zur Behandlung - einschliesslich der Prävention und der Verzögerung des Fortschrittes - einer Krankheit ausgewählt aus der Gruppe Zahnverfall und Überempfindlichkeit der Zähne.

2. Eine Verbindung der Formel (I) gemäss Anspruch 1 worin R¹ iso-Propyl bedeutet.

3. Eine Zusammensetzung enthaltend
a) eine oder mehrere Verbindungen der Formel (I) gemäss Anspruch 1 oder 2,
b) eine oder mehrere wasserfreie Alkohole der Formel HOR² worin R² ein C₁ bis C₈ Alkyl, C₁ bis C₈ Hydroxyalkyl bedeutet,
c) optional pharmazeutisch akzeptable Additive,
zur Verwendung in einem Verfahren zur Behandlung - einschliesslich der Prävention und der Verzögerung des Fortschrittes - einer Krankheit ausgewählt aus der Gruppe Zahnverfall und Überempfindlichkeit der Zähne.

4. Eine Zusammensetzung gemäss Anspruch 3 worin R¹ und R² identisch sind.

5. Die Verbindung gemäss Anspruch 1, wobei besagter Zahnverfall Karies ist.

6. Die Zusammensetzung gemäss Anspruch 3, wobei besagter Zahnverfall Karies ist.

7. Verwendung einer Verbindung der Formel (I)
Ti(OR¹)₄ (I)
worin R¹ Ethyl oder iso-Propyl bedeutet,
für die kosmetische Behandlung der Zähne, ausgewählt aus der Gruppe umfassend Aufhellen der Zähne und Glasieren der Zähne.

8. Die Verwendung gemäss Anspruch 7, worin R¹ iso-Propyl bedeutet.

9. Verwendung einer Zusammensetzung enthaltend
a) eine oder mehrere Verbindungen der Formel (I) gemäss Anspruch 7 oder 8,
b) eine oder mehrere wasserfreie Alkohole der Formel HOR² worin R² ein C₁ bis C₈ Alkyl, C₁ bis C₈ Hydroxyalkyl bedeutet,
c) optional pharmazeutisch akzeptable Additive,
für die kosmetische Behandlung der Zähne, ausgewählt aus der Gruppe umfassend Aufhellen der Zähne und Glasieren der Zähne.

10. Die Verwendung gemäss Anspruch 9, worin R¹ und R² identisch sind.

11. Die Verwendung gemäss einem der Ansprüche 7 bis 9 worin besagte kosmetische Behandlung das Aufhellen der Zähne ist.

12. Die Verwendung gemäss einem der Ansprüche 7 bis 9 worin besagte kosmetische Behandlung das Glasieren der Zähne ist.

## Revendications

1. Un composé de formule (I)
Ti(OR¹)₄ (I)
R¹ représentant éthyle ou isopropyle,
pour une utilisation dans un procédé de traitement - incluant prévention et retard de progression - d'un désordre sélecté du groupe constitué d'une décomposition dentaire et d'une hyper sensibilité dentaire.

2. Un composé de la formule (I) selon la revendication 1, R¹ représentant isopropyle.

3. Une composition contenant
a) un ou plusieurs composés de la formule (I) selon la revendication 1 ou 2
b) un ou plusieurs alcools sans eau de la formule HOR², R² représentant un C₁ à C₈ alkyle, C₁ à C₈ hydroxyalkyle,
c)optionnellement des additives pharmaceutiquement acceptables,
pour une utilisation dans un procédé de traitement - incluant prévention et retard de progression - d'un désordre sélecté du groupe constitué d'une décomposition dentaire et d'une hyper sensibilité dentaire.

4. Une composition selon la revendication 3, R¹ et R² étant identiques.

5. Le composé selon la revendication 1, la décomposition dentaire étant des carries.

6. La composition selon la revendication 3, la décomposition dentaire étant des carries.

7. Utilisation d'un composé de formule (I)
Ti(OR¹)₄ (I)
R¹ représentant éthyle ou isopropyle,
pour le traitement cosmétique de dents, sélecté du groupe constitué d'un blanchiment de dents et d'une application d'une nouvelle couche dentaire.

8. Utilisation selon la revendication 7, R¹ représentant isopropyle.

9. Utilisation d'une composition contenant
a) un ou plusieurs composés de la formule (I) selon la revendication 7 ou 8;
b) un ou plusieurs alcools sans eau de la formule HOR², R² représentant un C₁ à C₈ alkyle, C₁ à C₈ hydroxyalkyle,
c)optionnellement des additives pharmaceutiquement acceptables,
pour le traitement cosmétique de dents, sélecté du groupe constitué d'un blanchiment de dents et d'une application d'une nouvelle couche dentaire.

10. La composition selon la revendication 9, R¹ et R² étant identiques.

11. L'utilisation selon l'une des revendications 7 à 9, le traitement cosmétique étant le blanchiment de dents.

12. La composition selon l'une des revendications 7 à 9, le traitement cosmétique étant l'application d'une nouvelle couche dentaire.
